# EUROPEAN PATENT APPLICATION

(11) **EP 4 049 694 A1**
(43) Date of publication of application: **31.08.2022**
(21) Application number: 21159122.7
(22) Date of filing: 25.02.2021
(51) Int. Cl.: A61M 1/06

(54) **STORING AND RELEASING ENERGY IN A BREAST PUMP DEVICE**

(71) Applicant: Koninklijke Philips N.V., 5656 AG Eindhoven (NL)
(72) Inventor: DOBRUSSKIN, Christoph, 5656 AE Eindhoven (NL); ATNAFU AMRA, Eyob, 5656 AE Eindhoven (NL); HESEN, Rob, 5656 AE Eindhoven (NL)
(74) Representative: Philips Intellectual Property & Standards

(57) **Abstract**

A pump assembly (10) that is configured to be used in a breast pump device is connectable to at least one expression unit of the breast pump device and comprises a pump arrangement (11) configured to realize a pressure profile to be applied to a breast during a milk extraction process to be performed by the breast pump device. The pump arrangement (11) comprises a pump line (12) including a pump element (14) and a pump load arrangement (13), and the pump arrangement (11) further comprises an energy storage unit (16) that is configured to receive and mechanically store energy from the pump line (12) and release energy to the pump line (12), respectively. The pump assembly (10) may be controlled such that the pump element (14) is caused to run continuously throughout operation of the pump assembly (10).

## Description

### FIELD OF THE INVENTION

The invention relates to a pump assembly that is configured to be used in a breast pump device comprising at least one expression unit configured to interact with a breast from which milk is to be extracted by means of the breast pump device, the pump assembly being connectable to the at least one expression unit and comprising a pump arrangement configured to realize a pressure profile to be applied to a breast during a milk extraction process when the pump assembly is actually used in the breast pump device and the breast pump device is operated to perform such a process, wherein the pump arrangement comprises a pump line including a pump element and a pump load arrangement that is drivable by the pump element to create low pressure strokes in the pressure profile.

Also, the invention relates to a breast pump device comprising at least one expression unit configured to interact with a breast from which milk is to be extracted by means of the breast pump device, and a pump assembly as mentioned.

Further, the invention relates to a method of operating a pump assembly that is connectable to at least one expression unit of a breast pump device, which expression unit is configured to interact with a breast from which milk is to be extracted by means of the breast pump device, wherein the pump assembly comprises a pump arrangement configured to realize a pressure profile to be applied to a breast during a milk extraction process when the pump assembly is used in the breast pump device and the breast pump device is operated to perform such a process, and wherein the pump arrangement comprises a pump line including a pump element and a pump load arrangement that is drivable by the pump element to create low pressure strokes in the pressure profile, the method comprising operating the pump arrangement to realize the pressure profile.

Still further, the invention relates to a controller that is configured to be used in a pump assembly that is connectable to at least one expression unit of a breast pump device, which expression unit is configured to interact with a breast from which milk is to be extracted by means of the breast pump device, wherein the pump assembly comprises a pump arrangement configured to realize a pressure profile to be applied to a breast during a milk extraction process when the pump assembly is used in the breast pump device and the breast pump device is operated to perform such a process, and wherein the pump arrangement comprises a pump line including a pump element and a pump load arrangement that is drivable by the pump element to create low pressure strokes in the pressure profile, wherein the controller is configured to provide output for controlling operation of the pump arrangement.

### BACKGROUND OF THE INVENTION

In general, a breast pump device is a well-known tool for extracting milk from a breast of a lactating woman, or from two breasts simultaneously. Breast pump devices may be used in various situations, for example, if a baby or infant is physically not capable of extracting milk from the breast, or if a mother is separated from her baby or infant and the baby or infant is to be fed with breast milk at a later stage, by the mother or another person. Hence, breast pump devices are used by women to express breast milk at a convenient time, to be stored for later consumption by their/a baby or infant. Breast pump devices may also be helpful in a situation in which it is desired to stimulate and increase milk production in women with a low milk supply or to relieve pressure from engorged breasts.

A breast pump device typically includes one or two expression units. The breast pump device may be of an integral design, in which case the device may be suitable to be worn on the body, or the breast pump device may comprise one or two separate expression units commonly known as expression kits. Among other things, an expression unit may include a breast-receiving funnel for receiving at least a part of a woman's breast and may be suitable for connection to a pump assembly comprising a pump arrangement for realizing an air pressure profile in the expression unit, by means of which milk expression from the breast is enabled. In practical cases, the pump arrangement is an electric vacuum pump arrangement, in which cases the pump element normally comprises an electric motor, but other types of electrically operated breast pump devices and manually operated breast pump devices are also known and used in practice. The fact is that by generating a pressure profile of a cyclic nature, also referred to as pressure cycle or vacuum cycle, possibly accompanied by a certain way of massaging the breast, a simulation of a feeding action is obtained, which triggers the necessary let-down reflex in the lactating woman using the breast pump device. For the sake of completeness, it is noted that the term "vacuum" as used in this text refers to a relatively low pressure, i.e. a pressure that is significantly lower than ambient pressure.

The invention is in the field of electrically operated breast pump devices and primarily relates to the pump assembly of such breast pump devices. The pump assembly is connectable to at least one expression unit and comprises the pump arrangement, which is configured to realize the air pressure profile that is applied to a breast during a milk extraction process. In particular, the pump arrangement comprises a pump line including a pump element and a pump load arrangement that is drivable by the pump element to create low pressure strokes in the pressure profile. Further, the pump assembly comprises a controller configured to control operation of the pump arrangement. It is commonly known for the controller of the pump assembly to be configured to control operation of the pump arrangement by driving the pump element with an on/off mechanism. An increase of a level of vacuum is obtained when the pump element is on, and a release of vacuum is possible when the pump element is off.

In practical cases, the breast pump device may comprise a vacuum unit that normally comprises an air system including an air inlet at the position of which the vacuum unit is connectable or connected to the expression unit, wherein the pump arrangement of the pump assembly may be part of the vacuum unit. In such cases, the pump arrangement can be used to suck air from the air system for the purpose of reducing the pressure and to thereby realize a higher level of vacuum. The pump arrangement may further comprise an air valve configured to let in ambient air to the air system for the purpose of allowing the pressure to increase and to thereby realize a lower level of vacuum or even a total release of vacuum.

A disadvantage related to the conventional configuration of the pump assembly and the conventional way of operating the pump arrangement is that the capacity of the pump element directly determines the maximum level of vacuum that can be realized by means of the pump arrangement. Thus, adapting the design of a pump assembly for enabling the pump arrangement to achieve a higher maximum level of vacuum conventionally requires equipping the pump arrangement with a more powerful pump element. Among other things, it is an object of the invention to provide a way of going against this design rule.

### SUMMARY OF THE INVENTION

The invention provides a pump assembly that is configured to be used in a breast pump device comprising at least one expression unit configured to interact with a breast from which milk is to be extracted by means of the breast pump device, the pump assembly being connectable to the at least one expression unit and comprising a pump arrangement configured to realize a pressure profile to be applied to a breast during a milk extraction process when the pump assembly is actually used in the breast pump device and the breast pump device is operated to perform such a process, wherein the pump arrangement comprises a pump line including a pump element and a pump load arrangement that is drivable by the pump element to create low pressure strokes in the pressure profile, and wherein the pump arrangement further comprises an energy storage unit configured to receive and mechanically store energy from the pump line and release energy to the pump line, respectively.

It follows from the above definition of the pump assembly according to the invention that the pump arrangement comprises an energy storage unit that is configured to receive and mechanically store energy from the pump line and release energy to the pump line, respectively. In this way, the capacity of the pump element can be even further employed. In particular, it may be so that the energy storage unit is couplable or coupled to the pump line for performing the respective functions of receiving and mechanically storing energy from the pump line and releasing energy to the pump line.

When a comparison is made between the conventional configuration of the pump arrangement and the configuration of the pump arrangement according to the invention, a number of notable advantages of the invention are found, which include the fact that a predetermined maximum level of vacuum can be achieved by means of a smaller pump element or a higher maximum level of vacuum can be achieved by means of the same pump element.

The energy storage unit may be of any suitable type. For example, the energy storage unit may be configured to store energy received from the pump line as kinetic energy, in which case the energy storage unit may comprise a flywheel, for example. According to another feasible option, the energy storage unit may be configured to store energy received from the pump element as potential energy, in which case the energy storage unit may comprise a torsion bar or a spring, for example.

In an embodiment of the pump assembly according to the invention, the pump line further includes a main interface arrangement configured to establish a coupling between the pump element and the pump load arrangement in a coupling setting thereof and to cause the pump element and the pump load arrangement to be decoupled from each other in a decoupling setting thereof. In that way, both coupling the pump element and the pump load arrangement to each other and decoupling the pump element and the pump load arrangement from each other are provided as practical possibilities, wherein it is noted that decoupling the pump element and the pump load arrangement from each other may be appropriate during a charging time of the energy storage unit. It may be advantageous for the main interface arrangement to comprise a suitable (sliding) clutch or the like.

When it comes to the position of the energy storage unit in the pump arrangement, various options exist as will be explained in the following.

In the first place, it may be so that the energy storage unit is arranged in the pump line. In such a case, assuming the presence of a main interface arrangement in the pump line as explained in the foregoing, it is possible for the energy storage unit to be couplable to the pump load arrangement through the main interface arrangement, in conjunction with the pump element. A position at which the energy storage unit is arranged in the pump line may be a position between the pump element and the pump load arrangement, for example, but another position of the energy storage unit is feasible as well.

In the second place, it may be so that the energy storage unit is arranged in an additional line that is parallel to the pump line. In such a case, it may be advantageous if the pump arrangement further comprises an energy store interface arrangement configured to establish a coupling between the energy storage unit and the pump line in a coupling setting thereof and to cause the energy storage unit to be decoupled from the pump line in a decoupling setting thereof, and an energy release interface arrangement configured to establish a coupling between the energy storage unit and the pump line in a coupling setting thereof and to cause the energy storage unit to be decoupled from the pump line in a decoupling setting thereof at another position on the pump line as the energy store interface arrangement. By having the energy store interface arrangement and the energy release interface arrangement as mentioned, it is possible to have different paths between the pump line and the additional line for energy to be stored in the energy storage unit and energy to be released from the energy storage unit.

It is practical if the pump assembly according to the invention comprises a controller configured to control operation of the pump arrangement. In respect of the above-mentioned option of having an energy store interface arrangement and an energy release interface arrangement in the pump arrangement, it is noted that the different paths between the pump line and the additional line for energy to be stored in the energy storage unit and energy to be released from the energy storage unit may be opened and closed at appropriate times. For example, the controller may be configured to alternately put the energy store interface arrangement to the coupling setting and the decoupling setting, and to alternately put the energy release interface arrangement to the coupling setting and the decoupling setting, particularly in such a way that the coupling setting of the energy store interface arrangement and the decoupling setting of the energy release interface arrangement overlap in time, and also the coupling setting of the energy release interface arrangement and the decoupling setting of the energy store interface arrangement overlap in time. Further, in respect of the above-mentioned option of having a main interface arrangement in the pump line, it may be practical if the controller is configured to alternately put the main interface arrangement to the coupling setting and the decoupling setting.

In the framework of the invention, it may be beneficial if the controller is configured to cause the pump element to run continuously throughout operation of the pump assembly, as this enables choosing another control mechanism than the conventional on/off mechanism in respect of the pump element. When a comparison is made between the conventional method of controlling the pump arrangement and the option of causing the pump element to run continuously throughout operation of the pump assembly, a number of notable advantages are found, which include the following: i) noise caused by the pump element during operation is of a more continuous character and thereby less disturbing, and ii) a level of vacuum to be reached during operation can be realized in less time as the process of reducing pressure by means of the pump element is initiated with the pump element already running at a certain speed instead of needing to increase speed from zero, which beneficial effect of the invention is even stronger on the basis of the fact that when the speed is increased from zero, initial friction needs to be overcome. Further, when the pump element is caused to run continuously throughout operation of the pump assembly, it is proposed to retrieve energy from the pump element in the periods between low pressure strokes instead of letting it go to waste, storing it and using it for the benefit of the low pressure strokes, so that the pump element can be even smaller and/or the maximum level of vacuum can be even higher and/or generation of vacuum can be done faster.

When the controller is configured to cause the pump element to run continuously throughout operation of the pump assembly, indeed, this does not necessarily mean that the pump element is made to run at a constant speed. In this respect, it is noted that it may be advantageous to allow reduction of a running speed of the pump element when the pump element is not needed for driving the pump load arrangement and/or when a valve or the like is opened to the environment to release pressure, for example.

The pump element may comprise an electric motor such as a motor that is normally used in an electric vacuum pump arrangement. Further, it is noted that the pump load arrangement may include a vacuum drawing mechanism, and also that the pump arrangement may further comprise an air valve configured to let in ambient air to the pump line.

The invention also relates to a breast pump device comprising at least one expression unit configured to interact with a breast from which milk is to be extracted by means of the breast pump device, and a pump assembly of the type in which the pump arrangement comprises a pump line, which pump line comprises a pump element and a pump load arrangement, and further comprises an energy storage unit, as explained in the foregoing. Various practical aspects which are commonly known in the field of breast pump devices are also possible in the context of the invention, such as the aspect of the expression unit comprising a funnel-shaped breast-receiving shield and an air outlet in air communication with the breast-receiving shield, wherein the pump assembly is connectable or connected to the expression unit at the position of the air outlet thereof, and the aspect of the expression unit comprising a milk outlet to which a milk-receiving container is connectable.

The invention further relates to a method of operating a pump assembly that is connectable to at least one expression unit of a breast pump device, which expression unit is configured to interact with a breast from which milk is to be extracted by means of the breast pump device, wherein the pump assembly comprises a pump arrangement configured to realize a pressure profile to be applied to a breast during a milk extraction process when the pump assembly is used in the breast pump device and the breast pump device is operated to perform such a process, and wherein the pump arrangement comprises a pump line including a pump element and a pump load arrangement that is drivable by the pump element to create low pressure strokes in the pressure profile, the method comprising i) operating the pump arrangement to realize the pressure profile, and ii) retrieving and mechanically storing energy from the pump line and releasing stored energy to the pump line during operation of the pump arrangement, respectively. Advantageously, the method also comprises operating the pump element of the pump arrangement of the pump assembly to run continuously throughout operation of the pump assembly.

The invention still further relates to a controller that is configured to be used in a pump assembly that is connectable to at least one expression unit of a breast pump device, which expression unit is configured to interact with a breast from which milk is to be extracted by means of the breast pump device, wherein the pump assembly comprises a pump arrangement configured to realize a pressure profile to be applied to a breast during a milk extraction process when the pump assembly is used in the breast pump device and the breast pump device is operated to perform such a process, wherein the pump arrangement comprises a pump line including a pump element and a pump load arrangement that is drivable by the pump element to create low pressure strokes in the pressure profile, and wherein the pump arrangement further comprises an energy storage unit configured to perform respective functions of receiving and mechanically storing energy from the pump line and releasing energy to the pump line, wherein the controller is configured to provide output for operating the energy storage unit to perform its respective functions. Advantageously, the controller is also configured to provide output for causing the pump element of the pump arrangement of the pump assembly to run continuously throughout operation of the pump assembly.

For the sake of completeness, it is noted that the various options relating to the controller as already addressed in the above description and explanation of the pump assembly are equally applicable when the invention is considered in the context of the controller as such. Further, the controller may include a communication arrangement for communicating with an external device or system that serves to provide relevant information about operation of a breast pump device in which the controller may be present and milk extraction processes performed by means of the breast pump device to a person, and that possibly also allows a person to provide the controller with input. Practical examples of such a device include a smartphone, a smartwatch, a tablet, a laptop and a computer.

The above-described and other aspects of the invention will be apparent from and elucidated with reference to the following detailed description of various embodiments of a pump assembly of a breast pump device.

### BRIEF DESCRIPTION OF THE DRAWINGS

The invention will now be explained in greater detail with reference to the figs., in which equal or similar parts are indicated by the same reference signs, and in which:
Fig. 1 diagrammatically shows a breast pump device comprising an expression unit, a vacuum unit, and a flexible hose interconnecting the expression unit and the vacuum unit;
Fig. 2 diagrammatically shows a number of components of a first embodiment of a pump assembly according to the invention, which is suitable for use in a breast pump device;
Fig. 3 diagrammatically shows a number of components of a second embodiment of a pump assembly according to the invention, which is suitable for use in a breast pump device; and
Fig. 4 shows a number of combinations of a graph of pump element speed against time and a graph of level of vacuum against time.

### DETAILED DESCRIPTION OF EMBODIMENTS

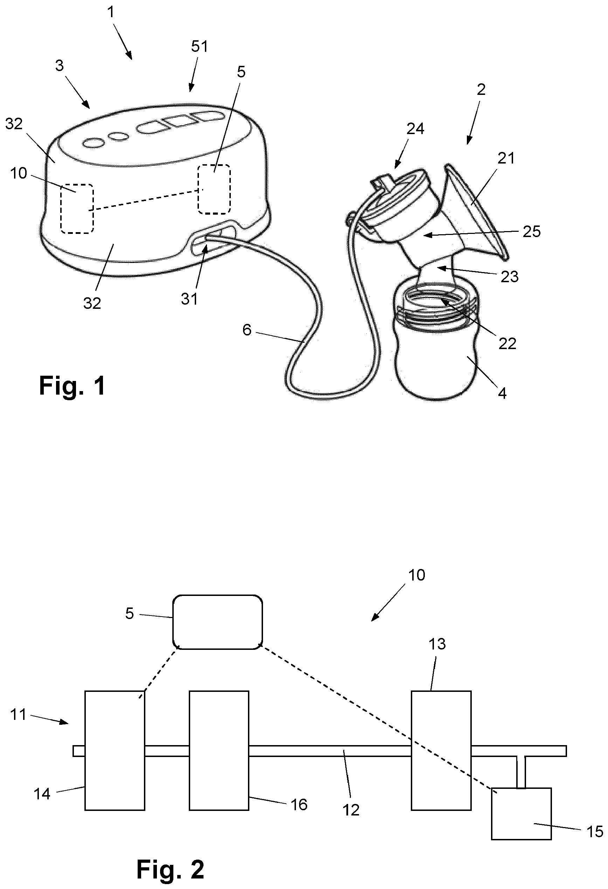

The invention is in the field of breast pump devices. With reference to fig. 1, a general description of an electric breast pump device will be given first so as to generate a clear picture of contextual aspects of the invention.

The breast pump device 1 comprises an expression unit 2 and a vacuum unit 3 for generating an air pressure cycle during which vacuum (low pressure) is alternately generated and released. For the purpose of receiving expressed breast milk during operation of the breast pump device 1, a milk receptacle 4 is used which is connectable to the expression unit 2, *e.g.* by screwing, thereby closing a lower end of the expression unit 2. The vacuum unit 3 is of an electric nature and comprises a pump assembly 10 for realizing an alternating vacuum during operation, i.e. during milk extraction processes to be performed by means of the breast pump device 1. The breast pump device 1 further comprises a controller 5 for realizing proper operation of the various components of the breast pump device 1.

At the level of the vacuum unit 3, the pump assembly 10 acts to realize the pressure cycle at the position of an air inlet 31 of an air system of the vacuum unit 3. The vacuum unit 3 may be electrically connectable to the mains or may include a battery or the like as a source of electric power. The pump assembly 10 and the controller 5 are diagrammatically depicted in fig. 1 as dashed rectangles. A path allowing for exchange of control signals etc. between the controller 5 and the pump assembly 10 is diagrammatically depicted in fig. 1 as a dashed line. The path may be realized as any suitable type of communication wire, for example. Fig. 1 depicts the controller 5 as being accommodated in a housing 32 of the vacuum unit 3, which is a practical option when it comes to the location of the controller 5.

The expression unit 2 comprises a breast-receiving funnel 21, an aperture acting as a milk outlet 22, and a milk path 23 from the breast-receiving funnel 21 to the milk outlet 22. The breast-receiving funnel 21 is thus in fluid communication with the milk outlet 22 through the milk path 23. The expression unit 2 further comprises an air outlet 24 and an air path 25 from the breast-receiving funnel 21 to the to the air outlet 24.

In fig. 1, the breast pump device 1 is shown in an assembled condition, in which the vacuum unit 3 is connected to the expression unit 2 through a flexible hose 6, wherein the air inlet 31 of the air system of the vacuum unit 3 is connected to the air outlet 24 of the expression unit 2 so that the pressure cycle that is generated by the pump assembly 10 during operation of the breast pump device 1 can be effective in the expression unit 2. The configuration including the flexible hose 6 for interconnecting the air inlet 31 of the air system of the vacuum unit 3 and the air outlet 24 of the expression unit 2 allows for a remote arrangement of the vacuum unit 3 with respect to the expression unit 2. It is to be noted that the breast pump device 1 can comprise two expression units 2 for enabling a lactating woman using the breast pump device 1 to extract milk from two breasts at the same time, in which case the expression units 2 can share a common vacuum unit 3.

In general, the breast pump device 1 is used to realize milk expression from a woman's breast. To that end, an alternating vacuum (pressure cycle) is applied to the breast. During periods of low pressure, or vacuum, the actual process in which milk is expressed from the breast takes place. Every time that the vacuum is released, freshly expressed milk flows in the receptacle 4.

Advantageously, as shown, the breast pump device 1 comprises a user interface 51 for allowing a user to control operation of the breast pump device 1. In the shown example, the user interface 51 is arranged on the housing 32 of the vacuum unit 3 and enables a user to provide input to the controller 5. The user interface 51 may be realized in any suitable manner such as through a number of buttons as shown, or through a touch screen, for example. By way of example, it is noted that the user interface 51 may comprise a button for activating a stimulation mode and a number of buttons for choosing one of a number of various expression settings.

Fig. 2 diagrammatically shows a number of components of a first embodiment of a pump assembly 10 according to the invention, which is suitable for use in a breast pump device 1. At one side, which is the right hand side in fig. 2, the pump assembly 10 is connectable to at least one expression unit 2 of a breast pump device 1. The pump assembly 10 comprises a pump arrangement 11 that is configured to realize an air pressure profile to be applied to a breast during a milk extraction process. The pump arrangement 11 comprises a pump line 12 including a pump load arrangement 13 and a pump element 14 for driving the pump load arrangement 13. The pump load arrangement 13 may be of any suitable type relying on any suitable pumping mechanism, and the pump element 14 may comprise a suitable electric motor or the like. Also, the pump arrangement 11 comprises an air valve 15 configured to let in air to the pump line 12, which air valve 15 may comprise a solenoid, for example.

In the first embodiment of the pump assembly 10 according to the invention, the controller 5 is configured to control the pump element 14 in such a way that the pump element 14 runs continuously throughout operation of the pump assembly 10. Also, the controller 5 is configured to alternately put the air valve 15 to an opened condition for reducing vacuum in the pump arrangement 11 and a closed position for not influencing the pressure in the pump arrangement 11. In that way, operation of the pump assembly 10 is obtained which causes the air pressure profile to be created with periods of generating low pressure strokes and periods of vacuum release in an alternating sequence.

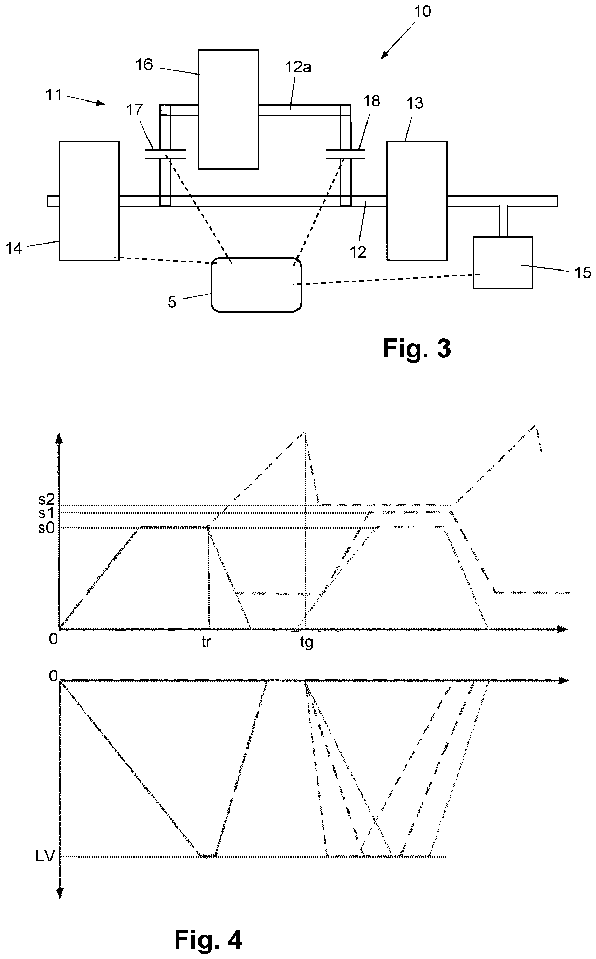

Fig. 2 illustrates the fact that in the embodiment shown, the pump arrangement 11 comprises an energy storage unit 16 such as a flywheel that is coupled to the pump element 14 at one side thereof for receiving and storing energy from the pump element 14, and that is coupled to the pump load arrangement 13 at another side thereof, for releasing stored energy to the pump load arrangement 13. The energy storage unit 16 serves to contribute to efficiency and stability of operation of the pump arrangement 11, and to help in realizing fast generation of vacuum when the air valve 15 is closed. During the time that the air valve 15 is in the opened condition, the energy storage unit 16 may cause the pump element 14 to keep running with no or little energy usage. It is noted that use of another type of energy storage unit than a flywheel is also possible in the context of the invention, wherein the pump assembly 10 may have a configuration similar to the one shown in fig. 2 or another configuration, such as the one that will be explained below on the basis of fig. 3.

Fig. 3 diagrammatically shows a number of components of a second embodiment of a pump assembly 10 according to the invention, which is suitable for use in a breast pump device 1. The second embodiment of the pump assembly 10 resembles the first embodiment of the pump assembly 10 to a considerable extent, comprising a pump arrangement 11 and a controller 5, wherein the pump arrangement 11 comprises a pump line 12 including a pump load arrangement 13 and a pump element 14 for driving the pump load arrangement 13. Also, the pump arrangement 11 of the second embodiment of the pump assembly 10 comprises an energy storage unit 16 that serves for receiving and storing energy from the pump element 14 and releasing stored energy to the pump load arrangement 13, but the arrangement of the energy storage unit 16 is in an additional line 12a that is parallel to the pump line 12.

In the pump arrangement 11 of the second embodiment of the pump assembly 10, the energy storage unit 16 is couplable to the pump line 12 at a position for receiving energy from the pump element 14. For the purpose of determining whether or not the energy storage unit 16 is coupled to the pump line 12 for receiving energy from the pump element 14 at a given point, the pump arrangement 11 further comprises an energy store interface arrangement 17 that is configured to establish a coupling between the pump line 12 and the energy storage unit 16 in a coupling setting thereof and to cause the pump line 12 and the energy storage unit 16 to be decoupled from each other in a decoupling setting thereof. Similarly, the pump arrangement 11 comprises an energy release interface arrangement 18 that is configured to establish a coupling between the pump line 12 and the energy storage unit 16 in a coupling setting thereof and to cause the pump line 12 and the energy storage unit 16 to be decoupled from each other in a decoupling setting thereof.

Basically, also in the second embodiment of the pump assembly 10 according to the invention, the controller 5 is configured to cause the pump element 14 to run continuously throughout operation of the pump assembly 10, as explained earlier. In the configuration of the pump arrangement 11 further comprising the energy store interface arrangement 17 and the energy release interface arrangement 18 mentioned in the foregoing, the controller 5 may be further configured in any suitable way to achieve both generation of an appropriate air pressure profile and optimal use of the energy storage unit 16. For example, the controller 5 may be configured to repeat a sequence of two steps in which a first step involves putting the energy store interface arrangement 17 to the decoupled setting and the energy release interface arrangement 18 to the coupled setting, so that the pump load arrangement 13 is driven by the pump element 14 and also receives energy from the energy storage unit 16 (provided that the sequence of steps has been performed at least one previous time), and a second step involves putting the energy store interface arrangement 17 to the coupled setting, and the energy release interface arrangement 18 to the decoupled setting, so that the energy storage unit 16 is charged with energy from the pump element 14. In this way, it is achieved that the pump load arrangement 13 is provided with an energy boost from both the pump element 14 and the energy storage unit 16 each time the first step is initiated, wherein it is to be noted that if the energy storage unit 16 would not be present in the pump arrangement 11, energy from the continuously running pump element 14 which would simply be lost. The action of opening the air valve 15 is intended to be performed at the time of the second step.

Fig. 4 shows a number of combinations of a graph of pump element speed against time, shown in the upper half of fig. 4, and a graph of level of vacuum against time, shown in the lower half of fig. 4. A first combination of graphs relates to conventional on/off control of the pump element 14. This first combination is indicated in a continuous line. A second combination of graphs relates to an option of causing the pump element 14 to run continuously without an energy storage unit 16 such as the flywheel addressed in the foregoing being present in the pump arrangement 11. This second combination is indicated in a relatively thick dashed line. A third combination of graphs relates to an option covered by the invention, namely the option of causing the pump element 14 to run continuously with an energy storage unit 16 such as the flywheel being present in the pump arrangement 11.

Among other things, it follows from a comparison from the three combinations of graphs that after an initial start-up of the pump arrangement 11, an intended maximum level of vacuum LV is reached faster when the pump element 14 is caused to run continuously, wherein the fastest generation of vacuum is associated with the option covered by the invention, i.e. the option of using an energy storage unit 16. Further, when the option of causing the pump element 14 to run continuously without using an energy storage unit 16 is compared to the conventional way of controlling the pump element 14, it is found that after the initial start-up, the level of pump element speed is at a somewhat higher level s 1 because there is no need to overcome initial friction. There is still a variation in pump element speed, wherein the pump element speed is alternately increased and reduced, but the reduction does not continue all the way to zero. Still further, when the option covered by the invention, i.e. the option of causing the pump element 14 to run continuously and using an energy storage unit 16 is considered, it is found that when the air valve 15 is opened at a vacuum release point tr, the pump element speed increases as the pump element 14 no longer needs to drive the pump load arrangement 13 and mainly outputs energy to the energy storage unit 16 instead. When the air valve 15 is closed again at a vacuum generation point tg, the pump element speed decreases. Assuming that the pump load arrangement 13 is also coupled to the energy storage unit 16 at that point, the pump element speed is reduced to a level s2 that is above both the conventional level s0 and the level s1 associated with the option of causing the pump element 14 to run continuously without using an energy storage unit 16.

On the basis of the advantageous effects of the option covered by the invention, following from causing the pump element 14 to run continuously throughout operation of the pump assembly 10 and also from additionally using an energy storage unit 16, it is possible to equip the pump arrangement 11 with a smaller pump element 14 and still achieve the same results when it comes to generating an air pressure profile, for example, or the leave the size of a pump element 14 of an existing design as it is and achieve higher levels of vacuum in a faster manner. In any case, when the pump element 14 is caused to run continuously, persons who are present to notice the sound generated by the pump arrangement 11 during operation thereof are not confronted with the irritating irregular sound profile of on/off operation of the pump element 14.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figs. and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figs., the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The term "comprise" as used in this text will be understood by a person skilled in the art as covering the term "consist of'. Hence, the term "comprise" may in respect of an embodiment mean "consist of', but may in another embodiment mean "contain/have at least the defined species and optionally one or more other species". The term "include" as used in this text is a synonym for the term "comprise" and will therefore be understood in a similar manner, i.e. so as to leave room for more species than just the one or ones explicitly referred to.

Notable aspects of the invention can be summarized as follows. A pump assembly 10 that is configured to be used in a breast pump device 1 is connectable to at least one expression unit 2 of the breast pump device 1 and comprises a pump arrangement 11 configured to realize a pressure profile to be applied to a breast during a milk extraction process to be performed by the breast pump device 1. The pump arrangement 11 comprises a pump line 12 including a pump element 14 and a pump load arrangement 13 that is drivable by the pump element 14 to create low pressure strokes in the pressure profile, and the pump arrangement 11 further comprises an energy storage unit 16 such as a flywheel that is configured to receive and mechanically store energy from the pump line 12 and release energy to the pump line 12, respectively. The pump assembly 10 may be controlled such that the pump element 14 is caused to run continuously throughout operation of the pump assembly 10.

## Claims

1. A pump assembly (10), configured to be used in a breast pump device (1) comprising at least one expression unit (2) configured to interact with a breast from which milk is to be extracted by means of the breast pump device (1), the pump assembly (10) being connectable to the at least one expression unit (2) and comprising a pump arrangement (11) configured to realize a pressure profile to be applied to a breast during a milk extraction process when the pump assembly (10) is actually used in the breast pump device (1) and the breast pump device (1) is operated to perform such a process, wherein the pump arrangement (11) comprises a pump line (12) including a pump element (14) and a pump load arrangement (13) that is drivable by the pump element (14) to create low pressure strokes in the pressure profile, and wherein the pump arrangement (11) further comprises an energy storage unit (16) configured to receive and mechanically store energy from the pump line (12) and release energy to the pump line (12), respectively.

2. The pump assembly (10) according to claim 1, wherein the energy storage unit (16) is configured to store energy received from the pump line (12) as kinetic energy.

3. The pump assembly (10) according to claim 1 or 2, wherein the energy storage unit (16) comprises a flywheel.

4. The pump assembly (10) according to any of claims 1-3, wherein the pump line (12) further includes a main interface arrangement configured to establish a coupling between the pump element (14) and the pump load arrangement (13) in a coupling setting thereof and to cause the pump element (14) and the pump load arrangement (13) to be decoupled from each other in a decoupling setting thereof.

5. The pump assembly (10) according to any of claims 1-4, wherein the energy storage unit (16) is arranged in the pump line (12).

6. The pump assembly (10) according to claim 5 insofar as dependent on claim 4, wherein the energy storage unit (16) is couplable to the pump load arrangement (13) through the main interface arrangement, in conjunction with the pump element (14).

7. The pump assembly (10) according to any of claims 1-4, wherein the energy storage unit (16) is arranged in an additional line (12a) that is parallel to the pump line (12).

8. The pump assembly (10) according to claim 7, wherein the pump arrangement (11) further comprises an energy store interface arrangement (17) configured to establish a coupling between the energy storage unit (16) and the pump line (12) in a coupling setting thereof and to cause the energy storage unit (16) to be decoupled from the pump line (12) in a decoupling setting thereof, and an energy release interface arrangement (18) configured to establish a coupling between the energy storage unit (16) and the pump line (12) in a coupling setting thereof and to cause the energy storage unit (16) to be decoupled from the pump line (12) in a decoupling setting thereof at another position on the pump line (12) as the energy store interface arrangement (17).

9. The pump assembly (10) according to any of claims 1-8, further comprising a controller (5) configured to control operation of the pump arrangement (11).

10. The pump assembly (10) according to claim 9 insofar as dependent on claim 8, wherein the controller (5) is configured to alternately put the energy store interface arrangement (17) to the coupling setting and the decoupling setting, and to alternately put the energy release interface arrangement (18) to the coupling setting and the decoupling setting, such that the coupling setting of the energy store interface arrangement (17) and the decoupling setting of the energy release interface arrangement (18) overlap in time, and also the coupling setting of the energy release interface arrangement (18) and the decoupling setting of the energy store interface arrangement (17) overlap in time.

11. The pump assembly (10) according to claim 9 or 10, wherein the controller (5) is configured to cause the pump element (14) to run continuously throughout operation of the pump assembly (10).

12. The pump assembly (10) according to any of claims 1-11, wherein the pump arrangement (11) further comprises an air valve (15) configured to let in ambient air to the pump line (12).

13. A breast pump device (1), comprising at least one expression unit (2) configured to interact with a breast from which milk is to be extracted by means of the breast pump device (1), and a pump assembly (10) according to any of claims 1-12.

14. Method of operating a pump assembly (10) that is connectable to at least one expression unit (2) of a breast pump device (1), which expression unit (2) is configured to interact with a breast from which milk is to be extracted by means of the breast pump device (1), wherein the pump assembly (10) comprises a pump arrangement (11) configured to realize a pressure profile to be applied to a breast during a milk extraction process when the pump assembly (10) is used in the breast pump device (1) and the breast pump device (1) is operated to perform such a process, and wherein the pump arrangement (11) comprises a pump line (12) including a pump element (14) and a pump load arrangement (13) that is drivable by the pump element (14) to create low pressure strokes in the pressure profile, the method comprising:
- operating the pump arrangement (11) to realize the pressure profile, and
- retrieving and mechanically storing energy from the pump line (12) and releasing stored energy to the pump line (12) during operation of the pump arrangement (11), respectively.

15. A controller (5), configured to be used in a pump assembly (10) that is connectable to at least one expression unit (2) of a breast pump device (1), which expression unit (2) is configured to interact with a breast from which milk is to be extracted by means of the breast pump device (1), wherein the pump assembly (10) comprises a pump arrangement (11) configured to realize a pressure profile to be applied to a breast during a milk extraction process when the pump assembly (10) is used in the breast pump device (1) and the breast pump device (1) is operated to perform such a process, wherein the pump arrangement (11) comprises a pump line (12) including a pump element (14) and a pump load arrangement (13) that is drivable by the pump element (14) to create low pressure strokes in the pressure profile, and wherein the pump arrangement (11) further comprises an energy storage unit (16) configured to perform respective functions of receiving and mechanically storing energy from the pump line (12) and releasing energy to the pump line (12), wherein the controller (5) is configured to provide output for operating the energy storage unit (16) to perform its respective functions.
